# EUROPÄISCHE PATENTSCHRIFT

(11) **EP 3 850 353 B1**
(45) Veröffentlichungstag und Bekanntmachung des Hinweises auf die Patenterteilung: **26.01.2022**
(21) Anmeldenummer: 19772970.0
(22) Anmeldetag: 10.09.2019
(51) Int. Cl.: G01N 29/04, G01N 25/72, G01N 29/30

(54) **VERFAHREN ZUR HERSTELLUNG EINES FASER-KUNSTSTOFF-VERBUND-VERGLEICHSKÖRPERS UND PRÜFUNGSVERFAHREN**
METHOD FOR PRODUCING A FIBRE-PLASTIC-COMPOSITE REFERENCE BODY AND TEST METHOD
PROCÉDÉ POUR PRODUIRE UN CORPS DE RÉFÉRENCE EN COMPOSITE FIBRES-MATIÈRE PLASTIQUE ET PROCÉDÉ DE CONTRÔLE

(30) Priorität: 10.09.2018 AT 507642018
(43) Veröffentlichungstag der Anmeldung: 21.07.2021
(73) Patentinhaber: FACC AG, 4910 Ried im Innkreis (AT)
(72) Erfinder: HÖLLER, Helmuth, 4910 Ried im Innkreis (AT)
(74) Vertreter: SONN Patentanwälte OG
(86) Internationale Anmeldenummer: PCT/AT2019/060291
(87) Internationale Veröffentlichungsnummer: WO 2020/051608

(56) Entgegenhaltungen:
- EP-A1- 2 431 736
- US-A1- 2014 272 324

## Beschreibung

Die Erfindung betrifft ein Verfahren zur Herstellung eines Faser-Kunststoff-Verbund-(FKV)-Vergleichskörpers zur Simulation einer Delamination für die zerstörungsfreie Prüfung von FKV-Bauteilen, insbesondere Flugzeug-Bauteilen.

Weiters betrifft die Erfindung ein Verfahren zur zerstörungsfreien Prüfung eines FKV-Bauteils, insbesondere Flugzeug-Bauteils.

Bei der Herstellung von sicherheitskritischen Faser-KunststoffVerbund-(FKV)-Bauteilen, wie beispielsweise Flugzeugbauteilen, ist die anschließende Überprüfung und Erfassung von Bauteilfehlern von besonderer Bedeutung. Zu diesem Zweck werden üblicherweise zerstörungsfreie Prüfverfahren (engl. "non-destructive testing", kurz NDT) eingesetzt, um einerseits fehlerhafte Bauteile sofort erkennen zu können und andererseits fehlerfreie Bauteile nicht durch das Prüfverfahren selbst zu beschädigen. Um bei den Prüfverfahren auch Rückschlüsse auf potentielle Fehlerquellen in der Produktion ziehen zu können, werden detektierte Bauteilfehler einer Fehlerart bzw. einer Fehlerklasse zugeordnet. Zu Vergleichs- und Kalibrierungszwecken werden dazu Vergleichskörper mit gezielt eingebrachten künstlichen Bauteilfehlern hergestellt und mit Hilfe eines NDT-Verfahrens vermessen. Um dabei die exakte Zuordnung eines Bauteilfehlers zu einer Fehlerklasse zu gewährleisten, müssen die als Referenz dienenden künstlichen Bauteilfehler in den Vergleichskörpern die Produktionsfehler der Prüflinge so präzise wie möglich nachbilden.

Die meisten Bauteilfehler sind ohne Hilfe von Fremdkörpern allerdings schwierig oder gar nicht nachzubilden und bislang weichen künstliche Bauteilfehler, je nach Fehlerart, in Hinblick auf die Beschaffenheit mehr oder weniger stark von den Fehlern der Prüflinge ab. Insbesondere eine sogenannte Delamination, d.h. eine lokale, flächige Trennung zweier FKV-Lagen in einem Teilbereich eines FKV-Bauteils unter Lufteinschluss, kann bislang ohne Fremdkörper einzubringen, nicht zufriedenstellend nachgebildet werden. Neben der Delamination existieren noch weitere Bauteilfehler, wozu insbesondere die Lagenporosität und die Volumenporosität zählen. Bei der Lagenporosität findet eine konzentrierte Ansammlung von mikroskopischen und makroskopischen Gas- bzw. Lufteinschlüssen in der Matrix bzw. dem Verbindungsmittel des FKV-Materials zwischen zwei FKV-Lagen eines Bauteils statt. Demnach liegt ein 2D-Bauteilfehler vor. Bei der Volumenporosität findet eine Ansammlung von Gas- bzw. Lufteinschlüssen in der Matrix bzw. dem Verbindungsmittel des FKV-Materials in mehreren FKV-Lagen eines Bauteils, insbesondere im Wesentlichen über den gesamten Querschnitt des FKV-Laminats, statt. Es handelt sich also um einen 3D-Bauteilfehler. Da eine Delamination, also eine flächige Trennung von FKV-Lagen, bei Flugzeugbauteilen schwerwiegende Folgen haben kann, ist das Erkennen einer Delamination und damit das Erzeugen von Vergleichskörpern mit möglichst realistischer Simulation der Delamination von hoher Wichtigkeit.

Aus dem Stand der Technik sind unterschiedliche Verfahren zum Nachbilden von Bauteilfehlern bekannt. In der EP 3 193 164 A1 wird beispielsweise ein Verfahren beschrieben, bei dem mit Hilfe eines Expansionskörpers Bauteilfehler in FKV-Teile eingebracht werden können. Dazu wird der Expansionskörper zwischen mehreren Lagen von FKV-Material gelegt, Harz hinzugefügt und anschließend erhitzt. Auf Grund des hohen Ausdehnungskoeffizienten des Expansionskörpers schrumpft dieser während des Abkühlens stärker zusammen als das ihn umgebende FKV-Material und erzeugt dadurch einen großen bleibenden Hohlraum. Der Expansionskörper verbleibt anschließend als Fremdkörper im Bauteil.

Bei der CN104407060 wird eine Porosität des Materials mit Hilfe von Glaskügelchen simuliert, die während des Produktionsprozesses in das Material eingebracht werden. Diese verbleiben allerdings ebenfalls im Material.

Des Weiteren sind aus der EP 1 750 123 A2, der EP 2 431 736 A1 und der US 2014/0272324 A1 weitere Verfahren zum Nachbilden von Bauteilfehlern bekannt.

Daneben ist aus der US 2014/0346405 A1 ein Verfahren zur Erzeugung von Porosität in Verbundwerkstoffen bekannt. Dazu werden die Verbundwerkstoffmaterialien unterschiedlichen Aushärteverfahren ausgesetzt, um so unterschiedliche Porositätsgrade durch entweichende Gase zu erzeugen.

Nachteilig beim Stand der Technik ist, dass eine Delamination, also eine flächige Trennung einzelner FKV-Lagen innerhalb eines FKV-Bauteils, nicht zufriedenstellend bzw. nicht ohne Fremdkörper nachgebildet werden kann. Unter anderem verfälschen dabei die Fremdkörper das Messergebnis.

Der Erfindung liegt somit die Aufgabe zugrunde, zumindest einzelne Nachteile des Standes der Technik zu lindern bzw. zu beseitigen. Die Erfindung setzt sich daher insbesondere zum Ziel, ein Verfahren zu schaffen, bei dem die realitätsnahe Nachbildung einer Delamination an definierten Stellen in einem FKV-Vergleichskörper ermöglicht wird.

Die gestellte Aufgabe wird dabei durch ein Verfahren mit zumindest folgenden Schritten gelöst:
i. Herstellen eines ersten Einsatzteils für den FKV-Vergleichskörper durch
   a. Anordnen einer ersten FKV-Lage;
   b. Ausbilden einer Vertiefung in der ersten FKV-Lage;
   c. Voraushärten der ersten FKV-Lage, um den ersten Einsatzteil für den FKV-Vergleichskörper zu erhalten;
ii. Herstellen eines zweiten Einsatzteils für den FKV-Vergleichskörper durch
   a. Anordnen einer zweiten FKV-Lage;
   b. Voraushärten der zweiten FKV-Lage, um den zweiten Einsatzteil für den FKV-Vergleichskörper zu erhalten;
iii. Vorsehen von zumindest einer ersten FKV-Schicht mit einer ersten Aussparung und zumindest einer zweiten FKV-Schicht mit einer zweiten Aussparung;
iv. Einsetzen des ersten Einsatzteils in die erste
   Aussparung der ersten FKV-Schicht und Einsetzen
   des zweiten Einsatzteils in die zweite Aussparung
   der zweiten FKV-Schicht;
v. Aushärten der Anordnung aus der ersten FKV-Schicht mit dem ersten Einsatzteil und der zweiten FKV-Schicht mit dem zweiten Einsatzteil, wobei an der Vertiefung in der ersten FKV-Lage des ersten Einsatzteils eine Delamination nachgebildet wird.

Vorteilhafterweise ermöglicht das erfindungsgemäße Verfahren das gezielte Einbringen einer (künstlichen) Delamination in einen aus FKV-Material bestehenden FKV-Vergleichskörper. Da mit Hilfe des erfindungsgemäßen Verfahrens die Delamination ohne Einführung eines Fremdkörpers, d.h. einer nicht aus FKV-Material bestehenden und am zu vergleichenden Bauteil nicht vorhandenen Substanz, erzeugt werden kann, ist der FKV-Vergleichskörper besonders geeignet, eine Delamination bei den zu prüfenden FKV-Bauteilen, insbesondere für die Luftfahrtindustrie, auf realitätsnahe Weise nachzubilden. Die nachgebildete Delamination kann unter anderem zu Kalibrierungszwecken verwendet werden, indem der FKV-Vergleichskörper einem NDT-Messverfahren, beispielsweise einem Thermografieverfahren, unterzogen wird. Aufgrund der realistischen Beschaffenheit der nachgebildeten Delamination eignen sich die vom Vergleichskörper gewonnenen Messergebnisse besonders gut als Vergleichs- bzw. Referenzwerte für die NDT-Prüfung von FKV-Bauteilen. Da bei der Erstellung des FKV-Vergleichskörpers auf die Einbringung von Fremdkörpern, d.h. nicht aus dem FKV-Material bestehende und am zu vergleichenden Bauteil nicht vorhandene Teile, verzichtet werden kann, können Messkurven des FKV-Vergleichskörpers aufgenommen werden, die mit großer Genauigkeit jenen von Bauteilen entsprechen, die eine "natürliche", d.h. bei der Serienproduktion entstandene, Delamination aufweisen. Eine Delamination ist dabei, wie eingangs erwähnt, eine lokale, flächige Trennung zweier FKV-Lagen in einem Teilbereich eines FKV-Bauteils unter Lufteinschluss. Im Unterschied dazu findet bei einer Lagenporosität lediglich eine teilweise Trennung der FKV-Lagen statt. Bei einer Volumenporosität findet die Trennung über mehrere FKV-Lagen unter Lufteinschluss statt.

Für die Zwecke der Offenbarung stellt eine FKV-Schicht ebenso eine FKV-Lage dar. Die einzelnen FKV-Lagen werden in dem FKV-Vergleichskörper - wie bei den zu prüfenden FKV-Bauteilen - vorzugsweise durch lose oder zu Geweben verbundene und mit Harz oder einem anderen Verbindungsmittel getränkte Fasern gebildet. Das Verbindungsmittel kann zur Verbindung der Fasern innerhalb einer FKV-Lage, zur Verbindung der FKV-Lagen untereinander und zur Verbindung der FKV-Schichten mit den vorausgehärteten Einsatzteilen dienen. Als FKV-Material für sämtliche FKV-Lagen können unter anderem CFK- (Kohlenstofffaserverstärkter Kunststoff), GFK- (Glasfaserverstärkter Kunststoff) oder Aramidfaser-Verbundwerkstoff, insbesondere zu Prepreg verarbeitete CFK-, GFK- bzw. Aramidfaser-Verbundwerkstoff, vorgesehen sein.

Die Delamination wird bei dem erfindungsgemäßen Verfahren durch das Einsetzen der (vorausgehärteten) Einsatzteile in die jeweils entsprechende Aussparung der FKV-Schichten und Aushärten der benachbarten Anordnung aus erster und zweiter FKV-Schicht, vorzugsweise unter Druck, erzeugt. Die erste und die zweite Aussparung werden zweckmäßigerweise mit einem Messer oder einem anderen Schneidewerkzeug in die erste bzw. zweite FKV-Schicht geschnitten und weisen vorzugsweise im Wesentlichen die Form bzw. Kontur des äußeren Randes des ersten bzw. des zweiten Einsatzteils auf. Das Verbindungsmittel zur Verbindung der Einsatzteile, wie beispielsweise Harz oder ein anderes Verbindungsmittel, ist bevorzugt bereits in den FKV-Schichten enthalten. Durch das Verbinden der ersten und zweiten FKV-Schicht und dem Aneinanderpressen der beiden FKV-Schichten samt den Einsatzteilen, insbesondere während des Aushärtens, wird das Verbindungsmittel durch den Kapillareffekt in einen engen, zwischen dem ersten und zweiten Einsatzteil liegenden Fügespalt bis zur Vertiefung gesogen und verbindet dadurch die beiden Einsatzteile in einem Randbereich außerhalb der Vertiefung miteinander und mit den FKV-Schichten. Demnach gelangt das Verbindungsmittel zur Verbindung der Einsatzteile von der FKV-Schicht zwischen die vorausgehärteten Einsatzteile.

An der Grenze zur Vertiefung bzw. dem eingeschlossenen Hohlraum kommt die Sogwirkung des Kapillareffekts schließlich auf Grund des größeren Abstandes der beiden Einsatzteile zueinander zum Erliegen und das Verbindungsmittel wird im Wesentlichen nicht in die Vertiefung gezogen. Somit ist die Vertiefung im Wesentlichen frei von Verbindungsmittel und die erste und zweite FKV-Schicht haften im Bereich der Vertiefung nicht aneinander, so dass die nachgebildete Delamination durch Aushärtung entstehen kann. In den Bereichen außerhalb der Vertiefung entsteht durch das eingesogene Verbindungsmittel eine form- und kraftschlüssige Verbindung zwischen den Einsatzteilen bzw. der ersten bzw. zweiten FKV-Schicht. Eine FKV-Schicht entspricht in Hinblick auf Materialbeschaffenheit im Wesentlichen einer FKV-Lage.

Nach dem Einsetzen der Einsatzteile in die jeweiligen Aussparungen und dem Verbinden der FKV-Schichten ist die Öffnung der Vertiefung des ersten Einsatzteils dem zweiten Einsatzteil zugewandt. Darüber hinaus ist es günstig, wenn die FKV-Schichten derart verbunden werden, dass die Aussparungen der FKV-Schichten im Wesentlichen deckungsgleich aneinander liegen. Vorzugsweise sind zu diesem Zweck die Aussparungen und die Einsatzteile im Wesentlichen gleich groß. Darüber hinaus ist wichtig, dass der erste Einsatzteil derart in die erste Aussparung eingesetzt wird, dass ein durch die Vertiefung des ersten Einsatzteils und den zweiten Einsatzteil begrenzter Hohlraum, die sogenannte Lufttasche, gebildet wird.

Bei dem erfindungsgemäßen Verfahren werden der erste und der zweite Einsatzteil in einem vorausgehärteten Zustand in die jeweiligen Aussparungen eingefügt. Vorausgehärtet bedeutet in diesem Zusammenhang, dass die beiden Teile soweit ausgehärtet werden, dass diese für die weiteren Verfahrensschritte ihre Form im Wesentlichen von selbst beibehalten. Zum Voraushärten können die ersten Aushärteschritte eines für das verwendete FKV-Material geeigneten Aushärteverfahrens verwendet werden, d.h. das Aushärteverfahren kann beendet werden, sobald die Teile so weit ausgehärtet sind, dass diese für die weiteren Verfahrensschritte ihre Form im Wesentlichen von selbst beibehalten. Nachdem die erste FKV-Schicht mit der zweiten FKV-Schicht verbunden und die Einsatzteile in die entsprechenden Aussparungen eingesetzt wurden, wird die Anordnung aus erster und zweiter FKV-Schicht vollständig ausgehärtet, wodurch die nachgebildete Delamination entsteht. Das Aushärten bedeutet in diesem Zusammenhang, dass die erste und zweite FKV-Schicht samt Einsatzteile und Verbindungsmittel vollständig ausgehärtet wird. Vorzugsweise weisen die erste und die zweite Aussparung im Wesentlichen die Form bzw. Kontur des äußeren Randes des ersten bzw. des zweiten Einsatzteils auf. Damit weisen auch die Einsatzteile im Wesentlichen die gleichen Maße wie die jeweiligen Aussparungen auf.

Um einen besonders realistischen FKV-Vergleichskörper beliebiger Dicke zu erzeugen, kann zumindest eine FKV-Grundlage auf der einen Seite der Anordnung aus erster und zweiter FKV-Schicht und/oder zumindest eine FKV-Abschlusslage auf der anderen Seite der Anordnung aus erster und zweiter FKV-Schicht vorgesehen werden. Durch das Anfügen einer beliebigen Anzahl an FKV-Grundlagen und/oder der FKV-Abschlusslagen kann ein FKV-Vergleichskörper beliebiger Dicke erzeugt werden. Die Anzahl an FKV-Abschlusslagen und FKV-Grundlagen kann dabei voneinander verschieden sein. Durch das erfindungsgemäße Verfahren kann der Hohlraum bzw. die künstliche Delamination somit beliebig tief innerhalb des FKV-Vergleichskörper an einer dafür vorgesehenen Stelle angeordnet werden. Je nach Anwendung können auch mehrere Delaminationen (Lufttaschen) vorgesehen werden. Die weiteren Lufttaschen können in derselben Weise wie oben geschildert, d.h. mittels erster und zweiter Einsatzteile, ausgebildet werden.

Um den Kapillareffekt zu begünstigen und um eine besonders realistische Delamination zu erzeugen, ist es vorteilhaft, wenn der erste Einsatzteil eine umlaufende und vorzugsweise im Wesentlichen ebene Randfläche um die Vertiefung aufweist. Umlaufend bedeutet in diesem Zusammenhang, dass die Vertiefung allseitig von der Randfläche umgeben ist. Dadurch kann von allen Seiten Verbindungsmittel gleichmäßig in den zwischen den beiden Einsatzteilen liegenden Fügespalt gesogen werden. Die Randfläche kann zweckmäßigerweise symmetrisch um die Vertiefung ausgebildet sein. Um die Randfläche an eine bestimmte Form anzupassen oder nicht benötigte Teilbereiche der Randfläche zu entfernen, kann vorgesehen sein, dass die Randfläche mit einem entsprechenden Schneidewerkzeug zumindest teilweise zugeschnitten wird.

Im Zuge der Erfindung hat sich ergeben, dass für die Einsatzteile besonders günstige Abmessungen existieren, mit denen der Kapillareffekt soweit reguliert werden kann, dass zum einen genügend Verbindungsmittel für eine ausreichende Verbindung der Einsatzteile in den Fügespalt gesogen wird und zum anderen einen Eintritt des Verbindungsmittels in den Hohlraum verhindert werden kann. Zu diesem Zweck ist es günstig, wenn die Randfläche symmetrisch um die Vertiefung ausgebildet ist und das Verhältnis der konstanten Breite der umlaufenden Randfläche zur Breite der durch die umlaufende Randfläche begrenzten Öffnungsquerschnittsfläche der Vertiefung im Wesentlichen zwischen 0,1 und 10, insbesondere im Wesentlichen zwischen 2 und 8, beträgt. Die Breite der umlaufenden Randfläche ist demnach bevorzugt breiter als die Öffnungsquerschnittsfläche. Diese Maße resultieren aus der Erkenntnis, dass bei einer zu breiten Randfläche zu wenig Verbindungsmittel in den Spalt zwischen den Einsatzteilen gesogen wird, während bei einer zu schmalen Randfläche nachteiligerweise Verbindungsmittel in den Hohlraum gelangt. Es hat sich in Versuchen darüber hinaus herausgestellt, dass das Verhältnis umso größer gewählt werden muss, je tiefer die Vertiefung in der Anordnung aus erster und zweiter FKV-Schicht zu liegenden kommt, d.h. je mehr FKV-Grundlagen/Abschlusslagen über oder unter der Vertiefung angeordnet sind. Die Breite der Öffnungsquerschnittsfläche bezieht sich auf die maximale Entfernung zweier Randpunkte der Öffnungsquerschnitttsfläche. Bei einer kreisförmigen Öffnungsquerschnittsfläche entspricht die Breite dem Durchmesser der Öffnungsquerschnittsfläche. Die Breite der umlaufenden Randfläche bezieht sich auf die maximale Entfernung zwischen dem inneren, an den an die Öffnungsquerschnittsfläche angrenzenden Rand der Randfläche, und dem äußeren, mit dem äußeren Rand des Einsatzteils zusammenfallenden Rand der Randfläche. Bei dieser Ausführungsform befindet sich die Vertiefung im Wesentlichen mittig am ersten Einsatzteil, sodass die Randfläche symmetrisch um die Vertiefung ausgebildet ist.

In einer bevorzugten Ausführungsform ist vorgesehen, dass die maximale Höhe der Vertiefung geringer als die Dicke der ersten FKV-Schicht, insbesondere geringer als die Dicken (d.h. Höhenerstreckungen) sämtlicher FKV-Schichten und FKV-Lagen, ist. Dadurch kann eine Ausbuchtung darüberliegender FKV-Schichten bzw. FKV-Lagen reduziert werden. In einer besonders bevorzugten Ausführungsform weisen sämtliche im FKV-Vergleichskörper verwendeten FKV-Lagen und FKV-Schichten im Wesentlichen dieselbe Lagendicke auf. Bei dieser Ausführungsform ist bevorzugt vorgesehen, dass die Höhe der Vertiefung geringer ist als die verwendete Lagendicke.

Bei einer bevorzugten Ausführungsvariante ist vorgesehen, dass die Vertiefung vor dem Voraushärten der ersten FKV-Lage durch Auflegen der ersten FKV-Lage auf einem Formwerkzeug ausgebildet wird. Dazu wird die erste FKV-Lage in einem unausgehärteten, d.h. formbaren Zustand, auf dem Formwerkzeug platziert. Durch Voraushärten verbleibt die Vertiefung anschließend in der ersten FKV-Lage und damit im ersten Einsatzteil.

Um die Vertiefung auf besonders einfache Weise auszubilden, ist in einer ersten Ausführungsform als Formwerkzeug ein Plattenteil, insbesondere ein Metallplättchen, vorgesehen. Dieses Metallplättchen wird nach dem Voraushärten der FKV-Lage wieder entfernt und kann vorteilhafterweise erneut verwendet werden.

Bei einer alternativen Ausführungsvariante ist als Formwerkzeug ein Vorsprung an einem Formenträger vorgesehen. Hierzu wird die erste FKV-Lage im unausgehärteten Zustand auf dem Formenträger und dem Vorsprung platziert und dadurch die Vertiefung ausgebildet. Nach dem Voraushärten wird die FKV-Lage von dem Formenträger abgenommen.

Um das Lösen der vorausgehärteten FKV-Lagen zu erleichtern, ist es vorteilhaft, wenn das Formwerkzeug vor dem Einsetzen in die Aussparung mit einem Trennmittel, insbesondere einem flüssigen Trennmittel oder einer Trennfolie, versehen wird. Dadurch kann das Formwerkzeug ohne Beschädigung der ersten bzw. zweiten FKV-Lage entfernt werden. Natürlich können auch andere Teile, wie beispielsweise Formenträger, mit einem solchen Trennmittel versehen sein, um sämtliche FKV-Bauteile leicht lösen zu können.

Bei einer bevorzugten Ausführungsform ist vorgesehen, dass in der zweiten FKV-Lage eine weitere Vertiefung ausgebildet wird. Vorteilhafterweise kann dadurch der Hohlraum und damit der Lufteinschluss der Delamination vergrößert werden. Weist der zweite Einsatzteil eine weitere Vertiefung auf, so wird der zweite Einsatzteil vorzugsweise derart in die zweite Aussparung eingesetzt, dass die Öffnung der weiteren Vertiefung des zweiten Einsatzteils dem ersten Einsatzteil zugewandt ist. Vorteilhafterweise sind die FKV-Schichten bzw. Einsatzteile so ausgerichtet, dass die Öffnungen der Vertiefungen im Wesentlichen gegenüber liegen und ein Luftvolumen in einem durch die Vertiefungen gebildeten Hohlraum miteinander einschließen. In einer besonders bevorzugten Form sind der erste und der zweite Einsatzteil im Wesentlichen ident ausgeführt. Sämtliche Aussagen und Fertigungsschritte in dieser Offenbarung, welche die Vertiefung des ersten Einsatzteils betreffen, sind auf die weitere Vertiefung im zweiten Einsatzteil anwendbar.

Der oben beschriebene FKV-Vergleichskörper kann für die NDT-Prüfung von FKV-Bauteilen verwendet werden.

Das Verfahren zur zerstörungsfreien Prüfung eines FKV-Bauteils, insbesondere Flugzeug-Bauteils, umfasst zumindest die folgenden Schritte:
- Herstellung eines Faser-Kunststoff-Verbund-(FKV)-Vergleichskörpers wie zuvor beschrieben;
- Prüfen des FKV-Bauteils mittels eines zerstörungsfreien Prüfverfahrens, beispielsweise eines Thermografieverfahrens; und
- Vergleichen von Messergebnissen aus dem zerstörungsfreien Prüfverfahren für das FKV-Bauteil mit Vergleichswerten von dem FKV-Vergleichskörper.

Die Erfindung wird nachstehend anhand von bevorzugten Ausführungsformen weiter erläutert.
Fig. 1 zeigt die Platzierung einer ersten FKV-Lage auf einem Formenträger zur Herstellung des ersten Einsatzteils.
Fig. 2 zeigt einen ersten bzw. zweiten Einsatzteil.
Fig. 3a-3b zeigt das Einsetzen der Einsatzteile in die jeweiligen FKV-Schichten.
Fig. 4a-4b zeigen jeweils zwei Einsatzteile im Querschnitt.
Fig. 5a-5c zeigt die Ausbildung einer Delamination.
Fig. 6 zeigt ein Flussdiagramm des erfindungsgemäßen Verfahrens zur Herstellung eines FKV-Vergleichskörpers in einer bevorzugten Ausführungsvariante.
Fig. 7 zeigt ein Flussdiagramm eines NDT-Prüfverfahren mit einem durch das erfindungsgemäße Verfahren gemäß den Ansprüchen 1 bis 10 hergestellten FKV-Vergleichskörper.

In den Figuren sind einzelne Verfahrensschritte zur Herstellung eines FKV-Vergleichskörpers 26 dargestellt, welcher bei der NDT-Prüfung von FKV-Bauteilen, wie beispielsweise Flugzeugflügel oder Flugzeugklappen, eingesetzt werden kann.

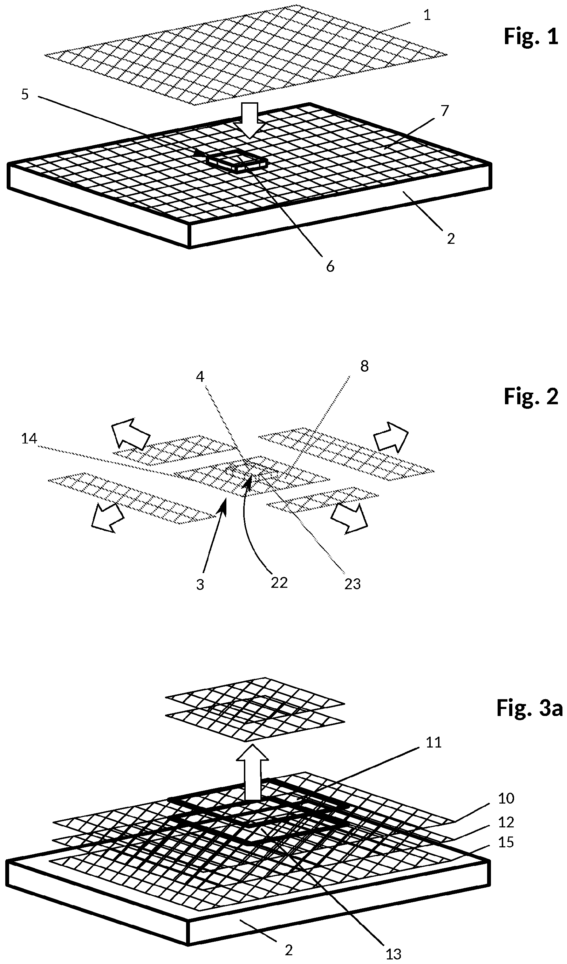

Fig. 1 zeigt eine (unausgehärtete) erste FKV-Lage 1, welche in Pfeilrichtung auf einem Formenträger 2 platziert wird, um einen ersten Einsatzteil 3 mit einer Vertiefung 4 (vgl. Fig. 2) herzustellen. Vorzugsweise besteht die erste FKV-Lage 1, wie vor allem bei Flugzeugbauteilen üblich, aus CFK-, GFK- oder Aramidfasern, insbesondere aus zu Prepreq verarbeiteten CFK-, GFK- bzw. Aramid-Materialien. Zur Erzeugung der Vertiefung 4 ist auf dem Formenträger 2 bevorzugt ein Formwerkzeug 5 in Form eines Vorsprunges 6 auf dem Formenträger vorgesehen. Durch Auflage der unausgehärteten ersten FKV-Lage 1 auf den Formenträger 2 passt sich diese im Wesentlichen an die Form des Formenträgers 2, insbesondere an den Vorsprung 6, an und bildet die Vertiefung 4 aus. An Stelle des Vorsprunges 6 kann auch beispielsweise ein Metallplättchen als Formwerkzeug 5 vorgesehen sein. Der Formenträger 2 gibt die spätere Form der (vor-)ausgehärteten FKV-Lagen vor, vorzugsweise eine im Wesentlichen ebene Fläche 7 wie in Fig. 1.

Nachdem die erste FKV-Lage 1 auf dem Formenträger 2 platziert und die Vertiefung 4 ausgebildet wurde, wird die erste FKV-Lage 1 durch entsprechende, dem Fachmann bekannte Verfahren vorausgehärtet, während das Formwerkzeug 5 in der ausgebildeten Vertiefung 4 verbleibt. Ein solches Voraushärteverfahren kann beispielsweise durch die ersten Schritte eines Aushärteverfahren in einem Autoklav (nicht gezeigt) durchgeführt werden. Durch das Voraushärten behält der erste Einsatzteil 3 seine Form für die weiteren Verfahrensschritte im Wesentlichen von selbst.

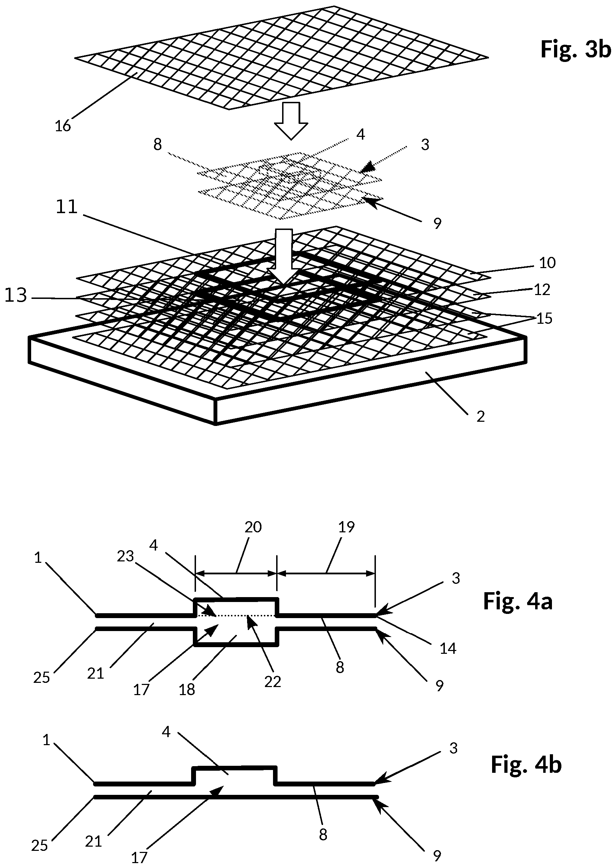

Fig. 2. zeigt einen vorausgehärteten ersten Einsatzteil 3 mit der Vertiefung 4 und einer umlaufenden, symmetrisch um die Vertiefung 4 ausgebildeten Randfläche 8. Die Vertiefung 4 ist im Wesentlichen mittig im ersten Einsatzteil 3 angeordnet und weist (auf der in Fig. 4a und Fig. 4b besser sichtbaren Unterseite des ersten Einsatzteils 3) eine Öffnung 22 und eine Öffnungsquerschnittsfläche 23, welche der Fläche der Öffnung 22 in der Ebene der Randfläche 8 entspricht, auf. Die Öffnungsquerschnittsfläche 23 wird durch die Randfläche 8 begrenzt. Durch die Pfeile wird angedeutet, dass die umlaufende Randfläche 8, beispielsweise durch ein geeignetes Schneidewerkzeug (nicht gezeigt), teilweise zugeschnitten und entfernt wird, um den Einsatzteil 3 anzupassen. Je nach Ausgangsmaterial ist das Zuschneiden der Randfläche 8 nicht zwingend erforderlich.

Um einen zweiten Einsatzteil 9 aus einer zweiten FKV-Lage 25 (vgl. Fig. 4a und Fig. 4b) zu erzeugen, können die gleichen Verfahrensschritte wie für den ersten Einsatzteil 3 durchgeführt werden. So kann ein zweiter Einsatzteil 9 erzeugt werden, der im Wesentlichen ident mit dem ersten Einsatzteil 3 ist. Es kann aber auch vorgesehen sein, dass der zweite Einsatzteil 9 keine Vertiefung aufweist, d.h. im Wesentlichen eben ausgebildet ist. Dazu kann die zweite FKV-Lage 25 ohne Formwerkzeug 5 auf einem Formenträger 2 platziert und vorausgehärtet werden. Der so gefertigte zweite Einsatzteil 9 besitzt eine im Wesentlichen plane Fläche und ist frei von Wölbungen.

Fig. 3a zeigt eine erste FKV-Schicht 10 mit einer ersten Aussparung 11 und eine zweite FKV-Schicht 12 mit einer zweiten Aussparung 13. Die Aussparungen 11, 13 werden dabei zweckmäßigerweise mit einem Messer oder einem anderen Schneidewerkzeug (nicht gezeigt) in die erste bzw. zweite FKV-Schicht 10, 12 geschnitten. Der Pfeil deutet erneut das Entfernen überschüssiger Abschnitte an. Die Aussparungen 11, 13 weisen für das spätere Einsetzen der Einsatzteile vorzugsweise die Form bzw. Kontur des äußeren Randes 14 des ersten 3 bzw. des zweiten Einsatzteils 9 auf. Weiters ist vorgesehen, dass die erste FKV-Schicht 10 derart mit der zweiten FKV-Schicht 12 verbunden wird, dass die Aussparung 11, 13 und damit später die Einsatzteile 3, 9 im Wesentlichen deckungsgleich übereinanderliegen. Vorzugsweise sind die Aussparungen 11, 13 und die Einsatzteile 3, 9 gleich groß, sodass die Einsatzteile 3, 9 bündig in die jeweiligen Aussparungen 11, 13 eingesetzt werden können.

Um die Fixierung der Einsatzteile 3, 9 zu verbessern und um einen besonders realistischen FKV-Vergleichskörper 26 zu erzeugen, bei dem die Delamination beliebig tief innerhalb des FKV-Vergleichskörpers 26 angeordnet ist, ist zumindest eine FKV-Grundlage 15 (in Fig. 3a ist eine FKV-Grundlage 15 und in Fig. 3b sind zwei FKV-Grundlagen 15 dargestellt) auf der einen Seite der Anordnung aus erster 10 und zweiter FKV-Schicht 12 vorgesehen. In Fig. 3b ist zudem auf der anderen Seite der Anordnung aus erster 10 und zweiter FKV-Schicht 12 zumindest eine FKV-Abschlusslage 16 vorgesehen. Die gesamte Anordnung ist zur späteren Aushärtung auf ebenfalls auf einem Formenträger 2 platziert. Die Pfeile deuten erneut das Zusammenfügen des späteren FKV-Vergleichskörpers an.

Fig. 3b zeigt das Einsetzen des ersten Einsatzteils 10 in die erste Aussparungen 11 der ersten FKV-Schicht 10 und das Einsetzen des zweiten Einsatzteils 9 in die zweite Aussparung 13 der zweiten FKV-Schicht 12. Wichtig ist dabei, dass der erste 3 und der zweite Einsatzteil 9 derart in die jeweiligen Aussparungen 11, 13 eingesetzt werden, dass ein durch die Vertiefung 4 des ersten Einsatzteiles 3 und den zweiten Einsatzteil 3 begrenzter Hohlraum 17, die sogenannte Lufttasche, gebildet wird, wie dies in den Figuren 4a und 4b ersichtlich ist. Die Öffnung 22 der Vertiefung 4 des ersten Einsatzteils 3 ist dabei dem zweiten Einsatzteil 9 zugewandt. Weist der zweite Einsatzteil 9 eine weitere Vertiefung 18 auf, so wird der zweite Einsatzteil 9 ebenfalls derart in die zweite Aussparung 13 eingesetzt, dass die Öffnung der weiteren Vertiefung 18 des zweiten Einsatzteils 9 dem ersten Einsatzteil 3 zugewandt ist. Wesentlich ist zudem, dass der erste 3 und der zweite Einsatzteil 9 bzw. die Aussparungen 11, 13 im Wesentlichen deckungsgleich aneinander liegen.

Fig. 4a zeigt den ersten Einsatzteil 3 und einen im Wesentlichen identen zweiten Einsatzteil 9 mit einer weiteren Vertiefung 18. Die beiden Einsatzteile 3, 9 sind derart angeordnet, dass ein durch die Vertiefungen des ersten und des zweiten Einsatzteils 3, 9 begrenzter Hohlraum 17 gebildet wird. Die Öffnung 22 der Vertiefung 4 des ersten Einsatzteils 10 ist dabei dem zweiten Einsatzteil 9 zugewandt, während die Öffnung der weiteren Vertiefung 18 des zweiten Einsatzteils 12 dem ersten Einsatzteil 10 zugewandt ist. Wie in Fig. 4a dargestellt, weist die umlaufende Randfläche 8 der Einsatzteile 3, 9 eine Breite 19 zwischen dem inneren, an die Öffnungsquerschnittsfläche 23 der Vertiefung 4 angrenzenden Rand und dem äußeren Rand 14 der Randfläche 8 bzw. des Einsatzteiles auf. Die Öffnungsquerschnittsfläche 23 der Vertiefung 4 wird durch die strichlierte Linie 23 angedeutet. Die durch die umlaufende Randfläche 8 begrenzte Öffnungsquerschnittsfläche 23 der Vertiefung 4 besitzt zudem eine Breite 20, welche sich bei nicht kreisrunden Öffnungsquerschnittsflächen 23 auf die maximale Breite bezieht. Das Verhältnis der Breite 19 der Randfläche 8 zur Breite 20 der Öffnungsquerschnittsfläche 23 beträgt vorzugsweise im Wesentlichen zwischen 0,1 und 10, insbesondere im Wesentlichen zwischen 2 und 8.

Fig. 4b zeigt eine Ausführungsform, bei der der zweite Einsatzteil 9 keine weitere Vertiefung aufweist. Der zweite Einsatzteil 9 ist im Wesentlichen flächig, d.h. frei von Wölbungen, ausgeführt.

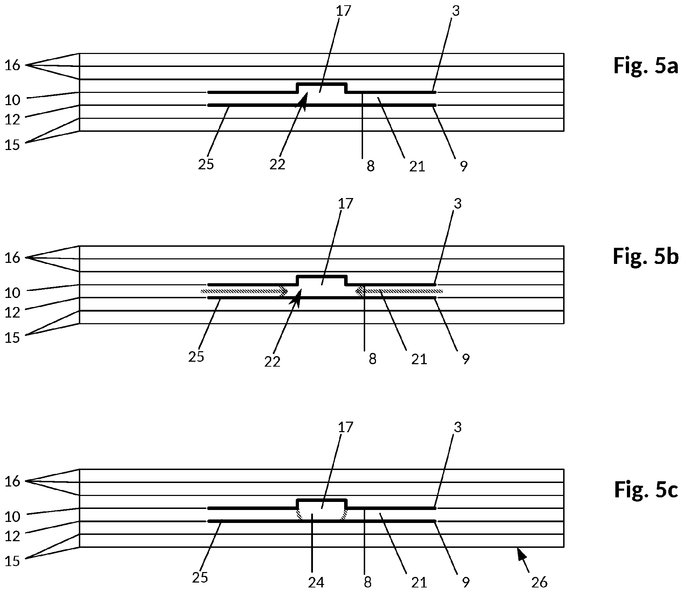

Fig. 5a zeigt die Anordnung aus der ersten FKV-Schicht 10 mit dem ersten Einsatzteil 3 und der zweiten FKV-Schicht 12 mit dem zweiten Einsatzteil 9. Durch das Aufwenden von Anpressdruck, insbesondere während eines für das FKV-Material geeigneten Aushärteverfahrens, wird durch den Kapillareffekt Verbindungsmittel, welches in dem verwendeten FKV-Material enthalten ist, in Pfeilrichtung in einen engen, zwischen dem ersten und zweiten Einsatzteil 3, 9 liegenden Fügespalt 21 bis zur Vertiefung gesogen und verbindet dadurch die beiden Einsatzteile 3, 9 in der Randfläche 8 außerhalb der Vertiefung 4 miteinander sowie mit der ersten 10 und zweiten FKV-Schichten 12. Demnach wird das Verbindungsmittel zur Verbindung der Einsatzteile 3, 9 von den FKV-Schichten zur Verfügung gestellt. An der Grenze zur Vertiefung 4 bzw. dem eingeschlossenen Hohlraum 17 kommt, wie in Fig. 5c dargestellt, die Sogwirkung des Kapillareffekts schließlich auf Grund des größeren Abstandes der beiden Einsatzteile 3, 9 zueinander im Wesentlichen zum Erliegen und das Verbindungsmittel wird nicht in die Vertiefung 4 bzw. den Hohlraum 17 gezogen. Somit ist die Vertiefung 4 im Wesentlichen frei von Verbindungsmittel und der erste und zweite Einsatzteil 3, 9 haften im Bereich der Vertiefung 4 nicht aneinander. Dadurch wird die nachgebildete Delamination 24 nach Aushärtung in der Vertiefung 4 bzw. im Hohlraum 17 gebildet.

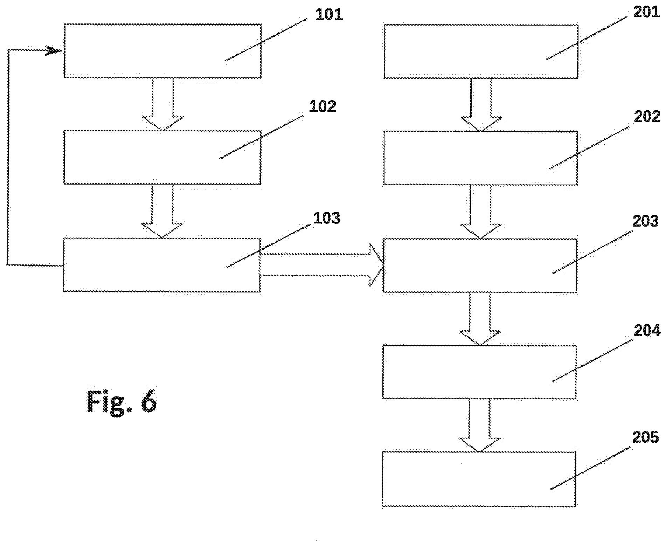

Fig. 6 zeigt einen bevorzugten Verfahrensablauf zur Herstellung eines FKV-Vergleichskörpers 26 mit einer künstlich erzeugten Delamination 24. In einem Schritt 101 wird zur Erzeugung eines Einsatzteils eine erste FKV-Lage 1 im unausgehärteten Zustand auf einem Formenträger 2 aufgelegt. Zur Ausbildung einer Vertiefung 4 kann ein Formwerkzeug 5 in Form eines Vorsprunges 6 auf dem Formenträger 2 bzw. in Form eines Metallplättchens vorgesehen sein. In einem Schritt 102 wird die erste FKV-Lage 1 vorausgehärtet, um den ersten Einsatzteil 3 zu erzeugen. In einem Schritt 103, der allerdings nicht zwingend notwendig ist, kann der erste Einsatzteil 3 auf eine bestimmte Größe zugeschnitten werden. Durch die Schritte 101-103 wird demnach der erste Einsatzteil 3 erzeugt. Durch nochmaliges Anwenden der Schritte 101-103 (wie durch den Pfeil angedeutet) können weitere Einsatzteile, insbesondere der zweite Einsatzteil 9, erzeugt werden. In einem Schritt 201 wird die erste 10 und die zweite FKV-Lage 12 in einem unausgehärteten Zustand auf einem Formenträger 2 aufgelegt. In einem Schritt 202 werden die erste 11 und die zweite Aussparung 13 aus der ersten 10 bzw. zweiten FKV-Schicht 12 geschnitten. In einem Schritt 203 werden die der erste 3 und der zweite Einsatzteil 9 in die erste 11 bzw. zweite Aussparung 13 eingesetzt. In einem Schritt 204 können bei Bedarf auch zumindest eine FKV-Grundlage 15 auf der einen Seite der Anordnung aus erster 10 und zweiter FKV-Schicht 12 und/oder zumindest eine FKV-Abschlusslage 16 auf der anderen Seite der Anordnung aus erster 10 und zweiter FKV-Schicht 12 angefügt werden. In einem Schritt 205 wird die gesamte Anordnung aus erster 10 und zweiter FKV-Schicht 12, dem ersten 3 und zweiten Einsatzteil 9 und etwaigen FKV-Grundlagen 15 und/oder FKV-Abschlusslagen 16 durch ein für das verwendete FKV-Material geeignetes Aushärteverfahren vollständig ausgehärtet.

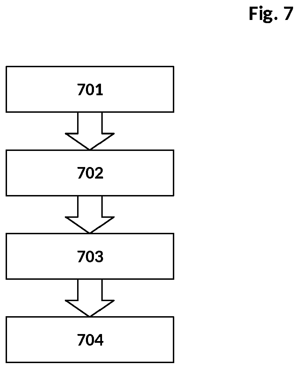

Fig. 7 zeigt einen bevorzugten Verfahrensablauf eines NDT-Prüfverfahrens mit einem FKV-Vergleichskörper 26. In einem Schritt 701 wird ein FKV-Vergleichskörper 26 durch den Verfahrensablauf gemäß Fig. 6 hergestellt. In einem Schritt 702 wird der Vergleichskörper mit einem zerstörungsfreien Prüfverfahren, beispielsweise einem Thermografieverfahren oder einem Ultraschallverfahren, geprüft, um die künstlich erzeugte Delamination 24 im FKV-Vergleichskörper 26 zu detektieren und zu vermessen. Dadurch können Vergleichswerte festgelegt werden. In einem Schritt 703 wird ein FKV-Bauteil aus FKV-Material, insbesondere ein Flugzeugbauteil, mit dem gleichen zerstörungsfreien Prüfverfahren geprüft, um Prüfergebnisse zu erhalten. In einem Schritt 704 werden die Prüfergebnisse aus Schritt 703 mit den Vergleichswerten aus Schritt 702 verglichen, um so eine Bewertung des FKV-Bauteils in Bezug auf allfällige Bauteilfehler, insbesondere eine Delamination, vornehmen zu können. Zu diesem Zweck werden vorzugsweise Signalamplituden oder andere, durch das zerstörungsfreie Prüferverfahren erzeugte, Signalarten miteinander verglichen. Bei Überschreiten eines festgelegten Grenzwertes, der aus den Vergleichswerten abgeleitet werden kann, kann ein fehlerhaftes FKV-Bauteil erkannt werden.

## Patentansprüche

1. Verfahren zur Herstellung eines Faser-Kunststoff-Verbund-(FKV)-Vergleichskörpers (26) zur Simulation einer Delamination (24) für die zerstörungsfreie Prüfung von FKV-Bauteilen, insbesondere Flugzeug-Bauteilen, mit den Schritten:
i. Herstellen eines ersten Einsatzteils (3) für den FKV-Vergleichskörper (26) durch
a. Anordnen einer ersten FKV-Lage (1);
b. Ausbilden einer Vertiefung (4) in der ersten FKV-Lage (1) ;
c. Voraushärten der ersten FKV-Lage (1), um den ersten Einsatzteil (3) für den FKV-Vergleichskörper zu erhalten;
ii. Herstellen eines zweiten Einsatzteils (9) für den FKV-Vergleichskörper durch
a. Anordnen einer zweiten FKV-Lage (25);
b. Voraushärten der zweiten FKV-Lage (25), um den zweiten Einsatzteil (9) für den FKV-Vergleichskörper zu erhalten;
iii. Vorsehen von zumindest einer ersten FKV-Schicht (10) mit einer ersten Aussparung (11) und zumindest einer zweiten FKV-Schicht (12) mit einer zweiten Aussparung (13);
iv. Einsetzen des ersten Einsatzteils (3) in die erste Aussparung (11) der ersten FKV-Schicht (10) und Einsetzen des zweiten Einsatzteils (9) in die zweite Aussparung (13) der zweiten FKV-Schicht (12);
v. Aushärten der Anordnung aus der ersten FKV-Schicht (10) mit dem ersten Einsatzteil (3) und der zweiten FKV-Schicht (12) mit dem zweiten Einsatzteil (9), wobei an der Vertiefung (4) in der ersten FKV-Lage (1) des ersten Einsatzteils (10) eine Delamination nachgebildet wird.

2. Verfahren nach Anspruch 1, **dadurch gekennzeichnet, dass** zumindest eine FKV-Grundlage (15) auf der einen Seite der Anordnung aus erster (10) und zweiter FKV-Schicht (12) und/oder zumindest eine FKV-Abschlusslage (16) auf der anderen Seite der Anordnung aus erster (10) und zweiter FKV-Schicht (12) vorgesehen wird.

3. Verfahren nach einem der Ansprüche 1 oder 2, **dadurch gekennzeichnet, dass** der erste Einsatzteil (3) eine umlaufende und vorzugsweise im Wesentlichen ebene Randfläche (8) um die Vertiefung (4) aufweist.

4. Verfahren nach Anspruch 3, **dadurch gekennzeichnet, dass** die Randfläche (8) symmetrisch um die Vertiefung (4) ausgebildet ist und das Verhältnis der Breite (19) der umlaufenden Randfläche (8) zur Breite (20) der durch die umlaufende Randfläche (8) begrenzten Öffnungsquerschnittsfläche (23) der Vertiefung (4) im Wesentlichen zwischen 0,1 und 10, insbesondere im Wesentlichen zwischen 2 und 8 beträgt.

5. Verfahren nach einem der Ansprüche 1 bis 4, **dadurch gekennzeichnet, dass** die maximale Höhe der Vertiefung (4) geringer als die Dicke der ersten FKV-Schicht (10), insbesondere geringer als die Dicke sämtlicher FKV-Schichten und FKV-Lagen, ist.

6. Verfahren nach einem der Ansprüche 1 bis 5, **dadurch gekennzeichnet, dass** die Vertiefung (4) vor dem Voraushärten der ersten FKV-Lage (1) durch Auflegen der ersten FKV-Lage (1) auf einem Formwerkzeug (5) ausgebildet wird.

7. Verfahren nach Anspruch 6, **dadurch gekennzeichnet, dass** als Formwerkzeug (5) ein Plattenteil, insbesondere ein Metallplättchen, vorgesehen ist.

8. Verfahren nach Anspruch 6, **dadurch gekennzeichnet, dass** als Formwerkzeug (5) ein Vorsprung (6) an einem Formenträger (2) vorgesehen ist.

9. Verfahren nach einem der Ansprüche 6 bis 8, **dadurch gekennzeichnet, dass** das Formwerkzeug (5) vor Auflegen der ersten FKV-Lage (1) mit einem Trennmittel, insbesondere einem flüssigen Trennmittel oder einer Trennfolie, versehen wird.

10. Verfahren nach einem der Ansprüche 1 bis 9, **dadurch gekennzeichnet, dass** in der zweiten FKV-Lage (25) eine weitere Vertiefung (18) ausgebildet wird.

11. Verfahren zur zerstörungsfreien Prüfung eines FKV-Bauteils, insbesondere Flugzeug-Bauteils, mit den Schritten:
- Herstellung eines Faser-Kunststoff-Verbund-(FKV)-Vergleichskörpers (26) in einem Verfahren nach einem der Ansprüche 1 bis 10;
- Prüfen des FKV-Bauteils mittels eines zerstörungsfreien Prüfverfahrens, beispielsweise eines Thermografieverfahrens; und
- Vergleichen von Messergebnissen aus dem zerstörungsfreien Prüfverfahren für das FKV-Bauteil mit Vergleichswerten von dem FKV-Vergleichskörper (26).

## Claims

1. A method for producing a fiber-plastic composite (FPC) reference body (26) for simulating a delamination (24) for the nondestructive testing of FPC components, in particular aircraft components, with the following steps:
i. Producing a first insert (3) for the FPC reference body (26) by
a. arranging a first FPC layer (1);
b. forming a recess (4) in the first FPC layer (1);
c. precuring the first FPC layer (1) to obtain the first insert (3) for the FPC reference body;
ii. Producing a second insert (9) for the FPC reference body by
a. arranging a second FPC layer (25);
b. precuring the second FPC layer (25) to obtain the second insert (9) for the FPC reference body;
iii. Providing at least one first FPC film (10) with a first clearance (11) and at least one second FPC film (12) with a second clearance (13);
iv. Inserting the first insert (3) into the first clearance (11) of the first FPC film (10) and inserting the second insert (9) into the second clearance (13) of the second FPC film (12);
v. Curing the arrangement comprised of the first FPC film (10) with the first insert (3) and the second FPC film (12) with the second insert (9), wherein a delamination is simulated at the recess (4) in the first FPC layer (1) of the first insert (10).

2. The method according to claim 1, **characterized in that** at least one FPC base layer (15) is provided on the one side of the arrangement comprised of the first (10) and second (12) FPC film and/or at least one final FPC layer (16) is provided on the other side of the arrangement comprised of the first (10) and second (12) FPC film.

3. The method according to one of claims 1 or 2, **characterized in that** the first insert (3) comprises a circumferential and preferably essentially even peripheral surface (8) around the recess (4) .

4. The method according to claim 3, **characterized in that** the peripheral surface (8) is designed symmetrically around the recess (4), and that the ratio between the width (19) of the circumferential peripheral surface (8) and the width (20) of the opening cross sectional surface (23) of the recess (4), the opening cross sectional surface (23) being bordered by the circumferential peripheral surface (8), measures essentially between 0.1 and 10, in particular essentially between 2 and 8.

5. The method according to one of claims 1 to 4, **characterized in that** the maximum height of the recess (4) is smaller than the thickness of the first FPC film (10), in particular smaller than the thicknesses of all FPC films and FPC layers.

6. The method according to one of claims 1 to 5, **characterized in that** the recess (4) is formed by applying the first FPC layer (1) on a molding tool (5) before precuring the first FPC layer (1).

7. The method according to claim 6, **characterized in that** a plate part, in particular a small metal plate, is provided as the molding tool (5).

8. The method according to claim 6, **characterized in that** a projection (6) on a mold carrier (2) is provided as the molding tool (5) .

9. The method according to one of claims 6 to 8, **characterized in that**, prior to applying the first FPC layer (1), the molding tool (5) is provided with a release agent, in particular a liquid release agent or a separating foil.

10. The method according to one of claims 1 to 9, **characterized in that** another recess (18) is formed in the second FPC layer (25).

11. A method for the nondestructive testing of an FPC component, in particular an aircraft component, with the following steps:
- producing a fiber-plastic composite (FPC) reference body (26) in a method according to one of claims 1 to 10;
- testing the FPC component with a nondestructive test method, for example a thermographic method; and
- comparing measurement results from the nondestructive test method for the FPC component with reference values for the FPC reference body (26).

## Revendications

1. Procédé de fabrication d'un bloc de référence (26) en plastique renforcé de fibres (PRF) destiné à simuler un délaminage (24) pour le contrôle non destructif de composants en PRF, en particulier de composants d'avion, comprenant les étapes suivantes :
i. fabrication d'un premier insert (3) pour le bloc de référence en PRF (26), comprenant
a. la mise en place d'une première couche de PRF (1) ;
b. la formation d'un renfoncement (4) dans la première couche de PRF (1) ;
c. le durcissement préalable de la première couche de PRF (1) afin d'obtenir le premier insert (3) pour le bloc de référence en PRF ;
ii. fabrication d'un second insert (9) pour le bloc de référence en PRF, comprenant
a. la mise en place d'une seconde couche de PRF (25) ;
b. le durcissement préalable de la seconde couche de PRF (25) afin d'obtenir le second insert (9) pour le bloc de référence en PRF ;
iii. obtention d'au moins une première couche de PRF (10) comprenant un premier évidement (11) et d'au moins une seconde couche de PRF (12) comprenant un second évidement (13) ;
iv. insertion du premier insert (3) dans le premier évidement (11) de la première couche de PRF (10) et insertion du second insert (9) dans le second évidement (13) de la seconde couche de PRF (12) ;
v. durcissement de l'assemblage de la première couche de PRF (10) comprenant le premier insert (3) et de la seconde couche de PRF (12) comprenant le second insert (9), dans lequel un délaminage est simulé au niveau du renfoncement (4) dans la première couche de PRF (1) du premier insert (10).

2. Procédé selon la revendication 1, **caractérisé en ce qu'**au moins une couche de base de PRF (15) est prévue sur un côté de l'assemblage de la première (10) et de la seconde couche de PRF (12) et/ou au moins une couche finale de PRF (16) est prévue sur l'autre côté de l'assemblage de la première (10) et de la seconde couche de PRF (12).

3. Procédé selon l'une des revendications 1 et 2, **caractérisé en ce que** le premier insert (3) présente une surface de bord (8) périphérique et de préférence sensiblement plane autour du renfoncement (4).

4. Procédé selon la revendication 3, **caractérisé en ce que** la surface de bord (8) est formée symétriquement autour du renfoncement (4) et le rapport entre la largeur (19) de la surface de bord périphérique (8) et la largeur (20) de la surface de section d'ouverture (23) du renfoncement (4) délimitée par la surface de bord périphérique (8) est essentiellement compris entre 0,1 et 10, en particulier essentiellement entre 2 et 8.

5. Procédé selon l'une des revendications 1 à 4, **caractérisé en ce que** la hauteur maximale du renfoncement (4) est inférieure à l'épaisseur de la première couche de PRF (10), et en particulier inférieure à l'épaisseur de l'ensemble des couches de PRF.

6. Procédé selon l'une des revendications 1 à 5, **caractérisé en ce que** le renfoncement (4) est formé avant le durcissement préalable de la première couche de PRF (1) en plaçant la première couche de PRF (1) sur un moule de formage (5).

7. Procédé selon la revendication 6, **caractérisé en ce que** le moule de formage (5) est une plaque, en particulier une plaquette métallique.

8. Procédé selon la revendication 6, **caractérisé en ce que** le moule de formage (5) est une saillie (6) sur un porte-moule (2).

9. Procédé selon l'une des revendications 6 à 8, **caractérisé en ce que** le moule de formage (5) est enduit d'un agent de démoulage, en particulier d'un agent de démoulage liquide ou d'un film de démoulage, avant l'application de la première couche de PRF (1).

10. Procédé selon l'une des revendications 1 à 9, **caractérisé en ce qu'**un autre renfoncement (18) est formé dans la seconde couche de PRF (25).

11. Procédé de contrôle non destructif d'un composant en PRF, en particulier d'un composant d'avion, comprenant les étapes suivantes :
- fabrication d'un bloc de référence en plastique renforcé de fibres (PRF) (26) selon un procédé de l'une des revendications 1 à 10 ;
- contrôle du composant en PRF à l'aide d'un procédé de contrôle non destructif, par exemple un procédé de thermographie ; et
- comparaison des résultats de mesure issus du procédé de contrôle non destructif pour le composant en PRF avec des valeurs de référence du bloc de référence en PRF (26).
